# EUROPEAN PATENT APPLICATION

(11) **EP 4 772 164 A1**
(43) Date of publication of application: **08.07.2026**
(21) Application number: 24860371.4
(22) Date of filing: 27.08.2024
(51) Int. Cl.: A61K 8/68, A61Q 19/00

(54) **CERAMIDE MIXTURE FOR IMPROVING COMPATIBILITY**

(30) Priority: 31.08.2023 KR 20230115811; 14.08.2024 KR 20240108872
(71) Applicant: Amorepacific Corporation, Seoul 04386 (KR)
(72) Inventor: YI, Changgeun, Yongin-si, Gyeonggi-do 17074 (KR); CHOI, Joonho, Yongin-si, Gyeonggi-do 17074 (KR); YOU, Jaewon, Yongin-si, Gyeonggi-do 17074 (KR); LEE, Somi, Yongin-si, Gyeonggi-do 17074 (KR); HAN, Youngkyu, Yongin-si, Gyeonggi-do 17074 (KR); BYOUN, Kyounghee, Yongin-si, Gyeonggi-do 17074 (KR); YOON, Jinyoung, Yongin-si, Gyeonggi-do 17074 (KR)
(74) Representative: Germain Maureau
(86) International application number: PCT/KR2024/012769
(87) International publication number: WO 2025/048451

(57) **Abstract**

Disclosed herein is a ceramide mixture. In an aspect, the ceramide mixture of the present invention can effectively improve the compatibility and solubility of ceramide. In another aspect, the microparticles comprising the ceramide mixture of the present invention can have a skin barrier component composition having a structure closer to normal skin due to increased structural uniformity and increased long periodicity phase (LPP) and orthorhombic structure compared to the case of simply mixing skin barrier components, and allows for the minimal use of formulation raw materials that may cause side effects, such as solvents, surfactants, dissolution aids, and the like. Moreover, the skin barrier components within the particles are protected by a coating agent, reducing interactions with external components. In another aspect, when applied to an oil-in-water emulsion, the ceramide mixture composition helps the formation of liquid crystals compared to the use of ceramide alone, and thus it is expected to improve sensory properties and moisturizing effects, which are advantages due to the formation of liquid crystals.

## Description

### [Technical Field]

### [Cross-Reference to Related Application]

The present application claims the benefit and priority of a Korean Patent Application No. 10-2023-0115811, filed on August 31, 2023, and having a title of the invention of "Ceramide mixture for improving compatibility", and a Korean Patent Application No. 10-2024-0108872, filed on August 14, 2024, and having a title of the invention of "Ceramide mixture for improving compatibility", and the entire contents of all such documents are incorporated herein by reference in the present specification.

In the present specification, a ceramide mixture having improved compatibility is disclosed.

### [Background Art]

A skin barrier is one of important human-body organs performing a protective function of the skin. The skin barrier is composed of an intercellular lipid component of the skin having ceramides, cholesterol, and fatty acids as main components, and corneocytes into which skin cells are differentiated, and is described as "brick and mortar model", "sandwich model", or the like. The skin barrier (the intercellular lipid component of the skin) is known to serve as a main barrier role of harmful bacteria or substances introduced from the outside. Among these, ceramides occupy 30 to 50 wt% among the intercellular lipid component of the skin, and play an important role in a skin barrier function such as skin moisturization and protection.

In a human body, various ceramides are known to exist, and there are research results reported as 18 to 22 types. Ceramides have a structure in which a fatty acid part (fatty acid moiety) and a sphingoid part (sphingoid base moiety) are combined, and a fact that carbon lengths thereof are various is known. In addition, a ceramide composition becomes different according to a skin site or a health state, and a fact that the ceramide composition changes in various skin diseases such as atopy, psoriasis, Lamellar ichthyosis, Netherton syndrome, and Chanarin-Dorfman syndrome was revealed. A ceramide NP, also known as a ceramide 3, constitutes 22.1%, which is the highest ratio among ceramides of a human body, and a fact that the ceramide NP is insufficient in diseases such as atopy is known, and utilization of the ceramide NP such as a clinical trial of improving moisturizing power (or moisturizing effects) of a human body using the ceramide NP has been studied.

However, directly using a component of a human-body-derived ceramide structure (a natural ceramide) thereby caused problems of high cost and low stability and the like. In order to solve such problems, a pseudo-ceramide was developed, and a representative one is PC-104 (hydroxypropyl bispalmitamide MEA). PC-104 is a pseudo-ceramide developed around 1996, and cytotoxicity tests and skin safety were confirmed, and, in various human-body moisturizing tests, effects such as improvement of atopy symptoms, skin moisturization, and improvement of a skin moisture state were confirmed, and, in an experiment comparing the moisturizing power of PC-104 and ceramide NP, no difference between the two has been reported. Also, from after development until now, for about 20 years, PC-104 was used in cosmetics, and, in 2006, there is also a case in which PC-104 was utilized as a ceramide component of a medicinal moisturizing agent (medicated moisturizer) of USA FDA approval 510(k). Accordingly, PC-104 is a raw material having a degree of utilization and reliability similar to human-body-derived ceramides.

Until now, there were structural studies of skin barrier components containing ceramides, but a structural study of a skin barrier mimic using a pseudo-ceramide is in a state of almost none. Since a higher-dimensional arrangement structure of lipid bilayers also is an important element in a main barrier function of ceramides, a structural study of a lipid composition including ceramides also should be preceded. For example, transepidermal water loss (TEWL) was reported to be related to a degree of orthorhombic packing existing in a stratum corneum of human skin. Also, it is known that in diseases such as atopy and psoriasis having abnormality in the skin barrier, an orthorhombic (ORT) structure decreases and, rather than a long periodicity phase (LPP) structure, a short periodicity phase (SPP) structure increases, and thus such structural research is thought to be able to help also in strengthening moisturizing power of skin barrier components. Recently, in studies using Fourier-transformed infrared Spectroscopy (FTIR) and the like, a fact that a mixed form rather than ceramides alone is more similar to a human body is confirmed, and similarly, when a pseudo-ceramide is mixed with ceramides of various structures and a structure is examined, whether the mixed form is more suitable for a human body may be confirmed.

Meanwhile, since ceramides are poorly soluble components, limitations exist such as causing problems in utilizing ceramides in various formulations or making a high-concentration product. Up to now, formulation or carrier research related to ceramides usually used methods and the like of using dissolution aids such as various surfactants or higher alcohols and the like. There were studies using an oil-in-water emulsion (O/W emulsion), a microemulsion, a vesicle using a solubilizer, a liposome, nanoparticles, microparticles, and the like. However, among such methods, when a component capable of disturbing the skin barrier such as a dissolution aid or a surfactant is used, side effects of such components should be considered, and an influence given by a surfactant to the intercellular lipid component of the skin may even lead to delipidation. In addition, in clinical practice, a fact that skin becomes rough, corneum is lifted, and skin becomes dry, only by wiping skin with a cosmetic cotton pad wetted with a 4% sodium dodecyl sulfate (SDS) solution, was confirmed, and, in this case, a fact that composition of ceramides, cholesterol, and fatty acids of the stratum corneum changes is known. Accordingly, research on a formulation method not utilizing such dissolution aids is thought to be necessary.

Accordingly, the present inventors, as a result of efforts to develop a method capable of implementing a more stable ceramide carrier while reducing help of dissolution aids such as surfactants through compatibility research between ceramides, confirmed an effect of improving compatibility of a ceramide mixture, and completed the present invention by confirming a structural research of the ceramide carrier as a skin barrier and an influence on skin absorption.

### [Disclosure]

### [Technical Problem]

One object of the present invention is to provide a ceramide mixture having improved compatibility.

### [Technical Solution]

In order to achieve the above object, the present invention, in one aspect, provides a ceramide mixture including one or more kinds of ceramide NP and PC-104 (hydroxypropyl bispalmitamide MEA).

### [Advantageous Effects]

In one aspect, the ceramide mixture of the present invention includes one or more kinds of ceramide NP and PC-104 (hydroxypropyl bispalmitamide MEA), and thus may effectively improve compatibility and solubility of ceramides.

In another aspect, microparticles including the ceramide mixture of the present invention, as compared with a case of simply mixing skin barrier components, have increased structural uniformity, and a long periodicity phase and an orthorhombic structure are increased, thereby being able to have a skin barrier component composition of a structure closer to normal skin.

In still another aspect, microparticles including the ceramide mixture of the present invention, while being able to minimize use of formulation raw materials causing side effects, such as a solvent, a surfactant, and a dissolution aid, are able to allow skin barrier components inside particles to be protected by a coating agent, thereby being able to minimize interaction with components outside particles, and have an advantage of being stable also in an aspect of dynamic equilibrium, as compared with a carrier such as an emulsion or a liposome.

In still another aspect, microparticles including the ceramide mixture of the present invention may increase the formation of liquid crystals. That is, a ceramide mixture composition, when applied to an oil-in-water emulsion, helps the formation of liquid crystals as compared with use of ceramides alone, and thus improvement of a feeling of use (or sensory properties) and moisturizing power, which are advantages by the formation of liquid crystals, is also expected.

In still another aspect, a composition of the present invention includes surfactants or dissolution aids and the like, only in a minimal amount, and thus may be applied on sensitive skin or on atopic skin without irritation.

### [Description of Drawings]

FIG. 1 relates to a total enthalpy of a ceramide mixture.

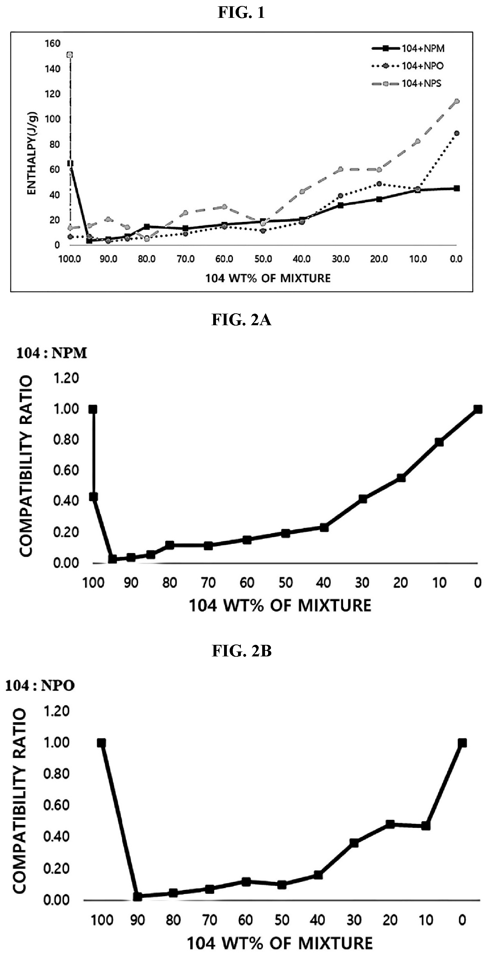

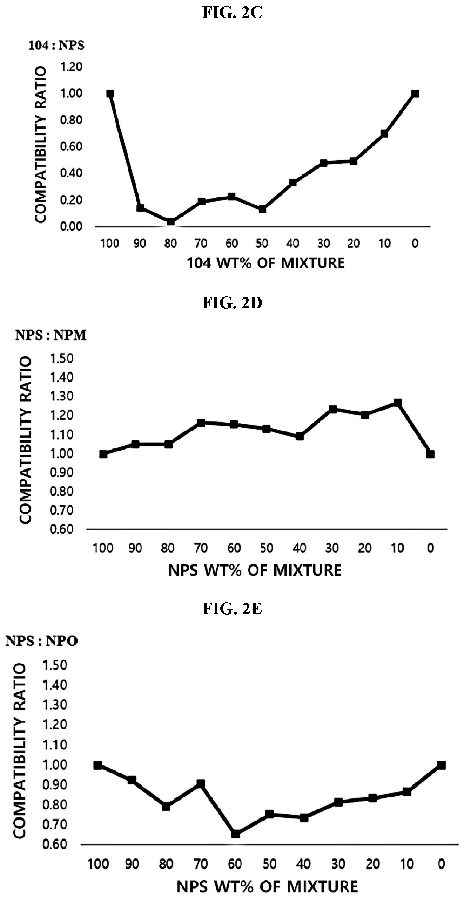

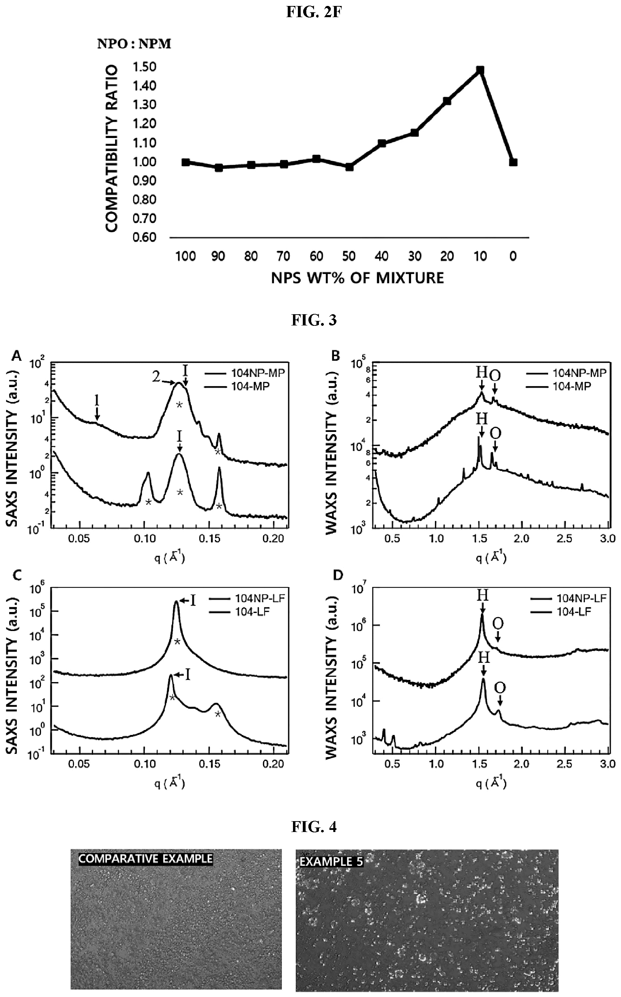

FIG. 2A to 2F relate to a ceramide compatibility ratio (abbreviated as ratio): (FIG. 2A) a 104 + NPM mixture (a region near 104 : NPM = 9 : 1 shows the lowest ratio), (FIG. 2B) a 104 + NPO (a region near 104 : NPO = 9 : 1 shows the lowest ratio, similar to the 104 + NPM mixture), (FIG. 2C) a 104 + NPS (a region near 104 : NPS = 8 : 2 shows the lowest ratio), (FIG. 2D) NPS + NPM (compatibility is not good; there is almost none of 1 or less), (FIG. 2E) NPS + NPO (a slightly good compatibility ratio is shown near NPS : NPO = 6 : 4, but is not good as compared with a mixture including 104), (FIG. 2F) NPO + NPM (compatibility is not good; there is almost none of 1 or less). Since enthalpy values obtained through differential scanning calorimetry (DSC) are different according to ceramides, in order to compare DSC values of mixtures between other ceramides, a calculation method of correcting the same is required. Accordingly, dividing a measured DSC enthalpy value by a theoretical enthalpy value calculated from DSC enthalpy values of two ceramide raw materials not mixed is defined as the ceramide compatibility ratio (or ratio), and the ceramide compatibility ratio is used.
FIG. 3A to 3D relate to small-angle X-ray scattering (SAXS) and X-ray diffraction (XRD) profiles of microparticles (MP) and lipid films (LF). All MP were swollen with a 0.15% aqueous solution of polyacrylic acid. In FIG. 3A to 3D, 1, 2: long periodicity phase (LPP), I: short periodicity phase (SPP), H: hexagonal, O: orthorhombic, *: compatibility are indicated.
FIG. 4 relates to an effect of increasing liquid crystals by ceramide mixing.

### [Mode for Disclosure]

Hereinafter, the disclosure will be described in detail.

In the present specification, "ceramide" includes not only a natural ceramide known to exist in a human body, in a form in which a fatty acid is bound to an amino group of a sphingoid skeleton, but also a pseudo-ceramide which is a compound having a chemical structure or a function similar to the natural ceramide.

In the present specification, the "ceramide NP" is a ceramide in which a sphingoid is phytosphingosine, and a fatty acid is a non-hydroxy fatty acid.

A product to which various raw materials and technologies intended to improve a state of skin by supplying skin barrier components to skin are applied is required. The present inventors, as a result of confirming compatibility between a pseudo-ceramide PC-104 and three kinds of ceramide NP, for development of formulation technology overcoming poor solubility of ceramides, which was one of conventional problems, confirmed that mixing between PC-104 and the ceramide NP is excellent in compatibility. In addition, by mixing mixed ceramide, stearic acid, and cholesterol to manufacture microparticles having a size of 380 µm or less, and through SAXS and WAXS analysis, it was confirmed that the microparticles have conditions more advantageous for making long periodicity phase (LPP) and an orthorhombic structure than when ceramides exist alone; or when ceramides, stearic acid, and cholesterol exist as a combination.

Accordingly, the present invention, in one viewpoint, relates to a ceramide mixture including one or more kinds of ceramide NP and PC-104 (hydroxypropyl bispalmitamide MEA).

The present invention, in another viewpoint, relates to a method of manufacturing a ceramide having improved compatibility, including a step of mixing one or more kinds of ceramide NP and PC-104 (hydroxypropyl bispalmitamide MEA).

In an exemplary implementation, the mixed ceramide may be manufactured through a method of simply mixing ceramides, a method of cooling after heating-and-dissolving ceramides together (melting/cooling), a method of removing a solvent after mixing-and-dissolving with help of the solvent, a method of milling-mixing in a Milling machine, a neat grinding method, and a grinding to which a liquid is added (liquid-assisted grinding) method, but is not limited thereto.

In an exemplary implementation, the ceramide NP may be characterized as being a ceramide NP to which a fatty acid having a carbon number of C10 to C24 is bound, but is not limited thereto.

In an exemplary implementation, the fatty acid may include one or more selected from the group consisting of lauric acid, myristic acid, palmitic acid, stearic acid, arachidic acid, behenic acid, and oleic acid, and, for example, as a combination of such fatty acids, the fatty acid may include lauric acid, myristic acid, palmitic acid, and stearic acid, and, in more detail, the fatty acid may be in a mixed form in which palmitic acid is mixed in 52-56 wt%, stearic acid is mixed in 42-47 wt%, myristic acid is mixed in at most 2 wt%, and lauric acid is mixed in at most 1 wt% or less, but is not limited thereto.

In an exemplary implementation, the ceramide NP may include one or more selected from the group consisting of a ceramide NP to which stearic acid is bound; a ceramide NP to which oleic acid is bound; and a mixed ceramide NP composed of a plurality of ceramide NP to which fatty acids having a carbon number of C14 to C24 are bound; but is not limited thereto.

In an exemplary implementation, PC-104 and the ceramide NP may be included in a weight ratio of 9.99:0.01 to 1:9.

In an exemplary implementation, the ceramide NP is a mixed ceramide NP composed of a plurality of ceramide NP to which fatty acids having a carbon number of C14 to C24 are bound, and PC-104 and the ceramide NP may be included in a weight ratio of 9.99:0.01 to 3:7, or 9.5:0.5 to 5:5. In more detail, in the above range, PC-104 may be 9.99 or less, 9.95 or less, 9.9 or less, 9.85 or less, 9.8 or less, 9.75 or less, 9.7 or less, 9.65 or less, 9.6 or less, 9.55 or less, or 9.5 or less, or may be 3 or more, 3.25 or more, 3.5 or more, 3.75 or more, 4 or more, 4.25 or more, 4.5 or more, 4.75 or more, or 5 or more. In addition, in the above range, the ceramide NP may be 0.01 or more, 0.05 or more, 0.1 or more, 0.15 or more, 0.2 or more, 0.25 or more, 0.3 or more, 0.35 or more, 0.4 or more, 0.45 or more, or 0.5 or more, or may be 7 or less, 6.75 or less, 6.5 or less, 6.25 or less, 6 or less, 5.75 or less, 5.5 or less, 5.25 or less, or 5 or less. For example, the weight ratio of PC-104 and the ceramide NP may be about 9.5:0.5, about 9:1, or about 8.5:1.5. Within the above range, compatibility of the ceramide mixture may be significantly improved.

In an exemplary implementation, the ceramide NP is a ceramide NP to which oleic acid is bound, and PC-104 and the ceramide NP may be included in a weight ratio of 9.99:0.01 to 1:9, or 9.99:0.1 to 4:6. In more detail, in the above range, PC-104 may be 9.99 or less, 9.95 or less, 9.9 or less, 9.85 or less, 9.8 or less, 9.75 or less, 9.7 or less, 9.65 or less, 9.6 or less, 9.55 or less, or 9.5 or less, or may be 1 or more, 1.25 or more, 1.5 or more, 1.75 or more, 2 or more, 2.25 or more, 2.5 or more, 2.75 or more, 3 or more, 3.25 or more, 3.5 or more, 3.75 or more, or 4 or more, and, also, in the above range, the ceramide NP may be 0.01 or more, 0.05 or more, 0.1 or more, 0.15 or more, 0.2 or more, 0.25 or more, 0.3 or more, 0.35 or more, 0.4 or more, 0.45 or more, or 0.5 or more, or may be 9 or less, 9.75 or less, 9.5 or less, 9.25 or less, 8 or less, 8.75 or less, 8.5 or less, 8.25 or less, 8 or less, 7.75 or less, 7.5 or less, 7.25 or less, 7 or less, 6.75 or less, 6.5 or less, 6.25 or less, or 6 or less. For example, a weight ratio of PC-104 and the ceramide NP may be about 9:1. Within the above range, compatibility of the ceramide mixture may be significantly improved.

In an exemplary implementation, the ceramide NP is a ceramide NP to which stearic acid is bound, and PC-104 and the ceramide NP may be included in a weight ratio of 9.99:0.01 to 2:8, or 9.99:0.1 to 5:5. In more detail, in the above range, PC-104 may be 9.99 or less, 9.95 or less, 9.9 or less, 9.85 or less, 9.8 or less, 9.75 or less, 9.7 or less, 9.65 or less, 9.6 or less, 9.55 or less, or 9.5 or less, or may be 2 or more, 2.25 or more, 2.5 or more, 2.75 or more, 3 or more, 3.25 or more, 3.5 or more, 3.75 or more, 4 or more, 4.25 or more, 4.5 or more, 4.75 or more, or 5 or more. Also, in the above range, the ceramide NP may be 0.01 or more, 0.05 or more, 0.1 or more, 0.15 or more, 0.2 or more, 0.25 or more, 0.3 or more, 0.35 or more, 0.4 or more, 0.45 or more, or 0.5 or more, or may be 8 or less, 7.75 or less, 7.5 or less, 7.25 or less, 7 or less, 6.75 or less, 6.5 or less, 6.25 or less, 6 or less, 5.75 or less, 5.5 or less, 5.25 or less, or 5 or less. For example, a weight ratio of PC-104 and the ceramide NP may be about 8:2. Within the above range, compatibility of the ceramide mixture may be significantly improved.

The present invention, in still another viewpoint, relates to a composition including the ceramide mixture.

In an exemplary implementation, the composition may further include ethanol as a solvent, but is not limited thereto.

In an exemplary implementation, fatty acids and/or cholesterol may be further included, but is not limited thereto.

In an exemplary implementation, the fatty acid may be a saturated fatty acid having a carbon number of 10 to 30, and, for example, the fatty acid may be one or more selected from the group consisting of Lauric acid, Myristic acid, Palmitic acid, Stearic acid, Arachidic acid, and Behenic acid, but is not limited thereto.

In an exemplary implementation, the composition may be characterized as having a form of particles or films, but is not limited thereto.

In an exemplary implementation, the composition may be an emulsion formulation, and the emulsion formulation may be an oil-in-water emulsion (O/W emulsion) formulation , but is not limited thereto.

In an exemplary implementation, the emulsion formulation may be characterized as having a liquid crystal structure, but is not limited thereto.

In an exemplary implementation, the particles may be nanoparticles.

In an exemplary implementation, the nanoparticles may further include various surfactants (for example, lecithin, polysorbate, and the like), and the nanoparticles may be manufactured through various manufacturing methods (for example, high-pressure emulsification, ultrasonic emulsification, chemical precipitation, spray drying, electrostatic spraying, mechanical milling, and the like).

In an exemplary implementation, a nanoparticle diameter (size) may be 10nm to 999nm, but is not limited thereto.

In an exemplary implementation, the particles may be microparticles.

In an exemplary implementation, a diameter (size) of the microparticles may be 400 µm or less, and, in more detail, may be 1 µm or more, 10 µm or more, 20 µm or more, 30 µm or more, 40 µm or more, 50 µm or more, 60 µm or more, 70 µm or more, 80 µm or more, 90 µm or more, 100 µm or more, 110 µm or more, 120 µm or more, 130 µm or more, 140 µm or more, 150 µm or more, 160 µm or more, 170 µm or more, 180 µm or more, 190 µm or more, 200 µm or more, 210 µm or more, 220 µm or more, 230 µm or more, 240 µm or more, or 250 µm or more, or may be 400 µm or less, 395 µm or less, 390 µm or less, 385 µm or less, 380 µm or less, 375 µm or less, 370 µm or less, 365 µm or less, 360 µm or less, or 355 µm or less. For example, a diameter (size) of the microparticles may be 380 µm or less, or 250 µm to 355 µm, but is not limited thereto. Within the above range, the microparticles may effectively load skin barrier components.

In an exemplary implementation, the microparticles may be an emulsified formulation made by mixing water and two or more kinds of substances that are not mixed with water, or two or more substances that are not mixed with each other, and a particle size may be from 1 µm or more to several hundred µm, but is not limited thereto.

In an exemplary implementation, the emulsified formulation may be water and vegetable oil, water and synthetic-derived oil, or water and silicone oil, or may be a mixture of silicone and oil not mixed with silicone, but is not limited thereto.

The present invention, in still another viewpoint, relates to a composition for skin moisturization; improvement of a skin moisture state; or prevention, improvement, or treatment of a skin disease, including the ceramide mixture.

The present invention, in still another viewpoint, relates to a cosmetic composition for skin moisturization; improvement of a skin moisture state; or prevention or improvement of a skin disease, including the ceramide mixture.

The present invention, in still another viewpoint, relates to a food composition for skin moisturization; improvement of a skin moisture state; or prevention or improvement of a skin disease, including the ceramide mixture.

The present invention, in still another viewpoint, relates to a quasi-drug composition for prevention or improvement of a skin disease, including the ceramide mixture.

The present invention, in still another viewpoint, relates to a pharmaceutical composition for prevention or treatment of a skin disease, including the ceramide mixture.

The present invention, in still another viewpoint, relates to a use of the ceramide mixture for manufacturing a composition for skin moisturization; improvement of a skin moisture state; or prevention, improvement, or treatment of a skin disease.

The present invention, in still another viewpoint, relates to a use of the ceramide mixture for skin moisturization; improvement of a skin moisture state; or prevention, improvement, or treatment of a skin disease.

In an exemplary implementation, the use is a therapeutic or non-therapeutic use.

The present invention, in still another viewpoint, relates to a method of skin moisturization; improvement of a skin moisture state; or prevention, improvement, or treatment of a skin disease, including a step of administering an effective amount of the ceramide mixture to a subject in need of skin moisturization; improvement of a skin moisture state; or prevention, improvement, or treatment of a skin disease.

In an exemplary implementation, the skin disease may be one or more selected from the group consisting of atopy, psoriasis, Lamellar ichthyosis, Netherton syndrome, and Chanarin-Dorfman syndrome, but is not limited thereto.

In an exemplary implementation, when skin barrier components are applied, damaged skin barrier may be improved to increase skin moisturization, and the same may be used also for purposes of general skin moisturization and improvement of a skin moisture state other than the skin disease.

In an exemplary implementation, the composition may include an active ingredient in 0.001 to 99.9 wt%. For example, the active ingredient may be included in 0.01 to 20.0 wt% or 0.1 to 10.0 wt%, but is not limited thereto.

In an exemplary implementation, the cosmetic composition may be manufactured in a form of a general emulsified formulation and a solubilized formulation. As the emulsified formulation, a nourishing toner, a cream, and an essence and the like are present, and, as the solubilized formulation, a softening toner and the like are present. A suitable formulation is not limited thereto, but, for example, may be in a form of a solution, a gel, a solid or paste anhydrous product, an emulsion obtained by dispersing an oil phase in an aqueous phase, a suspension, a microemulsion, a microcapsule, a microgranule or an ionic type (liposome), a non-ionic type vesicle dispersion agent, or in a form of a cream, a toner, a lotion, a powder, an ointment, a spray, or a concealer stick. In addition, the suitable formulation may be a form of a foam, or may be a form of an aerosol composition further containing a compressed propellant.

In an exemplary implementation, the cosmetic composition may additionally contain commonly used adjuvants such as fatty substances, organic solvents, solubilizers, thickeners and gelling agents, emollients, antioxidants, suspending agents, stabilizers, foaming agents, perfumes, surfactants, water, ionic or nonionic emulsifiers, fillers, metal ion sequestering agents, chelating agents, preservatives, vitamins, shielding agents, wetting agents, essential oils, dyes, pigments, hydrophilic or lipophilic active agents, lipid vesicles, or any other components commonly used in the cosmetic composition.

In an exemplary implementation, when the composition is used as an additive of a general food or a health functional food, the composition may be added as-is, or the composition may be used together with another food or a food ingredient, and the composition may be appropriately used according to a conventional method. A mixed amount of the active ingredient may be appropriately determined according to each use purpose such as prevention, health, or treatment. A food formulation may be possible as any of not only a form of a powder, a granule, a pill, a tablet, and a capsule, but also a form of a general food or a beverage.

In an exemplary implementation, a type of the general food or the health functional food is not particularly limited, and examples of foods to which the composition may be added include meat, confectioneries, noodles, gums, dairy products including ice creams, various soups, beverages, teas, drink preparations, alcohol beverages, and vitamin complexes and the like, and include all foods in a conventional meaning.

In an exemplary implementation, among the general food or the health functional food, a beverage may contain, as in a conventional beverage, various flavoring agents or natural carbohydrates and the like as additional components. The natural carbohydrates described above may be monosaccharides such as glucose and fructose, disaccharides such as maltose and sucrose, polysaccharides such as dextrin and sugar alcohols such as cyclodextrin, and xylitol, sorbitol, and erythritol. As a sweetener, a natural sweetener such as thaumatin and a stevia extract, or a synthetic sweetener such as saccharin and aspartame may be used. A ratio of the natural carbohydrates may be about 0.01 to 0.04 g, preferably about 0.02 to 0.03 g, per 100 mL of the beverage according to the present invention, but is not limited thereto.

In an exemplary implementation, in addition to the above, the general food or the health functional food according to the present invention may contain various nutrients, vitamins, electrolytes, flavoring agents, coloring agents, pectic acid and salts of pectic acid, alginic acid and salts of alginic acid, organic acids, protective colloid thickeners, pH adjusters, stabilizers, preservatives, glycerin, alcohol, and carbonation agents used in carbonated beverages. In addition, foods according to the present invention may contain fruit flesh for manufacturing natural fruit juice, fruit juice beverages, and vegetable beverages. Such components may be used independently or may be used in a mixed manner. A ratio of such additives is not limited, but is generally selected in a range of 0.01 to 0.1 parts by weight based on 100 parts by weight of the health functional food according to the present invention.

In an exemplary implementation, when the composition is used as a quasi-drug additive, the composition may be added as-is, or the composition may be used together with another quasi-drug or a quasi-drug ingredient, and the composition may be appropriately used according to a conventional method. A mixed amount of an active ingredient may be appropriately determined according to a use purpose. Components included in the quasi-drug composition, as active ingredients, may include components commonly used in a quasi-drug composition in addition to the active ingredient, and, for example, may include an abrasive, a wetting agent, a binder, a foaming agent, a sweetener, a preservative, a medicinal ingredient, a flavoring agent, a coloring agent, a solvent, a whitening agent, a solubilizer, or a pH adjuster.

In an exemplary implementation, the pharmaceutical composition may be provided in all formulations suitable for topical application. For example, the pharmaceutical composition may be administered by oral, transdermally, intravenous, intramuscular, or subcutaneous injection. As one example, the pharmaceutical composition may be an injection, an external-use solution for skin, a suspension, an emulsion, a gel, a patch, or a spray, but is not limited thereto. The formulation may be readily manufactured according to a conventional method in the art, and a surfactant, an excipient, a hydrating agent, an emulsification promoter, a suspending agent, a salt or a buffer for osmotic pressure control, a coloring agent, a spice, a stabilizer, a preservative, a conserving agent, or other commonly used adjuvants may be appropriately used.

In an exemplary implementation, an active ingredient of the pharmaceutical composition will vary according to an age, a gender, a weight, a pathological condition and severity thereof, an administration route, or a judgment of a prescriber of a subject. A determination of an appropriate dosage based on such factors is within a level of a person skilled in the art.

### [Mode for carrying out the invention]

Hereinafter, the configuration and effects of the present invention will be described in more detail by way of examples. However, the following examples are provided only for exemplary purposes to help understanding of the present invention, and a scope and range of the present invention are not limited thereby.

### [Examples]

### [Example 1]

### Evaluation of improvement of compatibility according to ceramide mixing

Ceramide compatibility was evaluated through measurement of differential scanning calorimetry (DSC). It is known that a ceramide compatibility study through DSC measurement may be utilized when measurement is not performed by polythermal and isothermal methods, or when slurry density of a solution is high, but has almost never been utilized in a ceramide study. In prediction of solubility of indomethacin (IMC) in polymers such as hydroxypropyl methylcellulose (HPMC) and polyvinylpyrrolidone (PVP), it was also confirmed that solubility prediction of a DSC method is excellent in a combination of IMC and HPMC. Since confirming compatibility between ceramides is a situation of confirming solubility when two solid-phase substances are mixed, a DSC experiment is determined to be very suitable, and thus the present inventors confirmed compatibility by measuring enthalpy of each ceramide, predicting enthalpy of a binary mixture, and actually measuring the same. The ceramides used are as shown in Table 1. For DSC measurement, a Thermal Analysis System (differential scanning calorimetry) such as Discovery DSC 250 of TA Instruments was used. In a DSC graph, melting enthalpy was measured by obtaining a peak area, and a unit of the peak area was J/g. A compatibility ratio was calculated by comparing a peak area measurement result of a sample in which ceramides are mixed and a calculated theoretical value (calculated sum area). A compatibility ratio is a ratio indicating compatibility when two components are mixed, and may be used to evaluate whether two components are stable even when mixed and may maintain desired characteristics.. The ratio is measured peak area / calculated theoretical value, and, when the value is 1, the same indicates that a peak area same as the theoretical value was measured, and, when the Ratio is 0.5, indicates that since an actually measured peak area is 1/2 of the theoretical value, the substance may be melted with 1/2 of energy of calculated energy. That is, as the value may be smaller than 1, a measured peak area as compared with a theoretical value is smaller, this means that a substance may be melted even with low energy, thereby indicating that compatibility is excellent.

**[Table 1]**

| **Used ceramides** | |
|---|---|
| **Sample** | **Ceramide type** |
| PC-104 | HYDROXYPROPYL BISPALMITAMIDE MEA |
| NPO | CERAMIDE NP(oleic acid) |
| NPS | CERAMIDE NP(stearic acid) |
| NPM | CERAMIDE NP(C_{14~24} fatty acid mixture) |

A single sample in which PC-104 and ceramide NP are unmixed (10:0 or 0:10), and samples in which PC-104 and ceramide NP are mixed at ratios such as 1:9 to 9.99:0.01, were each made, a DSC peak area was measured, and an actually measured DSC peak area and a calculated theoretical value according to a ratio of PC-104 and ceramide NP were compared. For compatibility evaluation, a ceramide mixing ratio having a low compatibility ratio was confirmed.

Melting enthalpy values of each ceramide are as shown in Table 2 below. PC-104 showed the highest value as 151.6 J/g, and the ceramide NP showed high enthalpy values in an order of NPS, NPO, and NPM. A ceramide has a structure in which a sphingoid skeleton and a fatty acid are bound, and NPS to which stearic acid is bound as the fatty acid showed higher enthalpy than NPO to which oleic acid is bound, and NPM is a ceramide raw material made using a fatty acid mixture having various lengths of carbon numbers of 14 to 24, and is a raw material in which ceramides of various structures are compounded, not a ceramide composed of a single structure. NPM showed the lowest value of a total enthalpy value among NP.

**[Table 2]**

| **Total melting enthalpy of used ceramides** | |
|---|---|
| **Product name** | **Melting enthalpy (J/g)** |
| PC-104 | 151.6 |
| NPS | 114.6 |
| NPO | 89.0 |
| NPM | 45.3 |

As illustrated in Table 3, it was confirmed that, in a range in which a weight ratio of PC-104:NPM is 9.99:0.01 to 3:7, ratio is 0.5 or less, and, particularly, in a range of 9.5:0.5 to 5:5, it was confirmed that ratio is 0.2 or less. That is, since energy required for melting is reduced to 1/2 or less by mixing two ceramides in a range in which a weight ratio of PC-104:NPM is 9.99:0.01 to 3:7, it was confirmed that compatibility is significantly improved. Particularly, when a weight ratio of PC-104:NPM is 9.5:0.5, 9:1, and 8.5:1.5, a similar level was shown as ratio 0.1 or less. Including PC-104 and NPM, ethanol solubility may be improved by 1.67 times as compared with a theoretical value, and may be improved by 25 times as compared with single use.

**[Table 3]**

| **DSC analysis results according to mixing ratio of PC-104 and NPM** | | | | |
|---|---|---|---|---|
| PC-104 | NPM | Peak area (J/g) | Calculated sum area (J/g) | Ratio |
| 10 | 0 | 151.6 | 151.6 | 1.00 |
| 9.99 | 0.01 | 65.4 | 151.5 | 0.43 |
| 9.5 | 0.5 | 3.9 | 146.3 | 0.03 |
| 9 | 1 | 4.9 | 141.0 | 0.03 |
| 8.5 | 1.5 | 7.2 | 135.7 | 0.05 |
| 8 | 2 | 15.0 | 130.4 | 0.12 |
| 7 | 3 | 13.6 | 119.7 | 0.11 |
| 6 | 4 | 16.6 | 109.1 | 0.15 |
| 5 | 5 | 19.0 | 98.5 | 0.19 |
| 4 | 6 | 20.4 | 87.8 | 0.23 |
| 3 | 7 | 32.0 | 77.2 | 0.41 |
| 2 | 8 | 36.8 | 66.6 | 0.55 |
| 1 | 9 | 44.0 | 55.9 | 0.79 |
| 0 | 10 | 45.3 | 45.3 | 1.00 |

Further, as illustrated in Table 4, in a case of the ceramide PC-104 and NPO, it was confirmed that, in a range in which a weight ratio of PC-104 and NPO is 9.99:0.01 to 1:9, ratio is lower than 0.5, and, particularly, in a range of 9.99:0.01 to 4:6, it was confirmed that ratio is 0.2 or less.

**[Table 4]**

| **DSC analysis results according to a mixing ratio of ceramide PC-104 and NPO** | | | |
|---|---|---|---|
| 104 | NPO | Peak area (J/g) | Ratio |
| 10 | 0 | 151.6 | 1.00 |
| 9.99 | 0.01 | 6.7 | 0.04 |
| 9.5 | 0.5 | 7.1 | 0.05 |
| 9 | 1 | 3.3 | 0.02 |
| 8.5 | 1.5 | 5.1 | 0.04 |
| 8 | 2 | 6.2 | 0.04 |
| 7 | 3 | 9.4 | 0.07 |
| 6 | 4 | 14.9 | 0.12 |
| 5 | 5 | 11.8 | 0.10 |
| 4 | 6 | 18.2 | 0.16 |
| 3 | 7 | 39.4 | 0.37 |
| 2 | 8 | 48.8 | 0.48 |
| 1 | 9 | 44.9 | 0.47 |
| 0 | 10 | 89.0 | 1.00 |

Further, as illustrated in Table 5, in a case of PC-104 and NPS, it was confirmed that, in a range in which a weight ratio of PC-104 and NPS is 9.99:0.01 to 2:8, ratio is 0.5 or less, and, particularly, in a range of 9.99:0.01 to 7:3 or 5:5, it was confirmed that ratio is 0.2 or less.

**[Table 5]**

| **DSC analysis results according to a mixing ratio of ceramide PC-104 and NPS** | | | |
|---|---|---|---|
| 104 | NPS | Peak area (J/g) | Ratio |
| 10 | 0 | 151.6 | 1.00 |
| 9.99 | 0.01 | 14.0 | 0.09 |
| 9.5 | 0.5 | 15.5 | 0.10 |
| 9 | 1 | 20.9 | 0.14 |
| 8.5 | 1.5 | 14.4 | 0.10 |
| 8 | 2 | 5.0 | 0.04 |
| 7 | 3 | 26.1 | 0.19 |
| 6 | 4 | 30.7 | 0.22 |
| 5 | 5 | 17.4 | 0.13 |
| 4 | 6 | 42.7 | 0.33 |
| 3 | 7 | 60.3 | 0.48 |
| 2 | 8 | 60.1 | 0.49 |
| 1 | 9 | 82.7 | 0.70 |
| 0 | 10 | 114.6 | 1.00 |

When a binary mixture was made, PC-104 generally showed good compatibility. In compatibility between PC-104 and NPS, NPO, and NPM, a compatibility ratio showed very low values as 0.04 to 0.7, 0.02 to 0.48, and 0.03 to 0.79, respectively. In mixing between NPS and NPO, a compatibility ratio was less than 1, thereby confirming improvement of compatibility, but a lowest value was 0.65, which was higher than when PC-104 was included. As illustrated in FIG. 2, a compatibility ratio between NPM and NPO was confirmed as 0.97 to 1.49, and a compatibility ratio between NPM and NPS was confirmed as 1.05 to 1.27. Therefore, in mixing between ceramides, it was able to be confirmed that including PC-104 is more advantageous than not including PC-104 in terms of improvement of enthalpy measured through DSC. Meanwhile, as illustrated in FIG. 1, mixing between PC-104 and a ceramide showed lowest values not only in a compatibility ratio but also in an enthalpy value. Specifically, an enthalpy value was shown as 67.7 J/g in mixing of NPS and NPO, and was shown as 45.3 J/g in mixing of NPS-NPM and NPO-NPM, and, in mixing of PC-104 and NPM, was shown as 3.9 J/g, in mixing of PC-104 and NPO was shown as 3.3 J/g, and in mixing of PC-104 and NPS was shown as 5.0 J/g (Table 6 and Table 7). Through such dramatic improvement of enthalpy of PC-104, a possibility of improvement of solubility of a PC-104 mixture was confirmed.

**[Table 6]**

| **Total melting enthalpy of mixed ceramides obtained in DSC test** | | | |
|---|---|---|---|
| | Enthalpy (J/g) | | |
| 104 Ratio | NPM | NPO | NPS |
| 100.0 | 151.6 | 151.6 | 151.6 |
| 99.9 | 65.4 | 6.7 | 14.0 |
| 95.0 | 3.9 | 7.1 | 15.5 |
| 90.0 | 4.9 | 3.3 | 20.9 |
| 85.0 | 7.2 | 5.1 | 14.4 |
| 80.0 | 15.0 | 6.2 | 5.0 |
| 70.0 | 13.6 | 9.4 | 26.1 |
| 60.0 | 16.6 | 14.9 | 30.7 |
| 50.0 | 19.0 | 11.8 | 17.4 |
| 40.0 | 20.4 | 18.2 | 42.7 |
| 30.0 | 32.0 | 39.4 | 60.3 |
| 20.0 | 36.8 | 48.8 | 60.1 |
| 10.0 | 44.0 | 44.9 | 82.7 |
| 0.0 | 45.3 | 89.0 | 114.6 |

**[Table 7]**

| **Final summary of compatibility ratio obtained from total melting enthalpy of ceramides obtained in DSC test mixed** | | | |
|---|---|---|---|
| | Compatibility ratio | | |
| 104 Ratio | 104+NPM | 104+NPO | 104+NPS |
| 100.0 | 1.00 | 1.00 | 1.00 |
| 99.9 | 0.43 | 0.04 | 0.09 |
| 95.0 | 0.03 | 0.05 | 0.10 |
| 90.0 | 0.03 | 0.02 | 0.14 |
| 85.0 | 0.05 | 0.04 | 0.10 |
| 80.0 | 0.12 | 0.04 | 0.04 |
| 70.0 | 0.11 | 0.07 | 0.19 |
| 60.0 | 0.15 | 0.12 | 0.22 |
| 50.0 | 0.19 | 0.10 | 0.13 |
| 40.0 | 0.23 | 0.16 | 0.33 |
| 30.0 | 0.41 | 0.37 | 0.48 |
| 20.0 | 0.55 | 0.48 | 0.49 |
| 10.0 | 0.79 | 0.47 | 0.70 |
| 0.0 | 1.00 | 1.00 | 1.00 |

### [Example 2]

### Evaluation of improvement of solubility according to ceramide mixing

Through Example 1, an effect of improvement of compatibility according to ceramide mixing confirmed through DSC measurement was confirmed. Accordingly, an effect of improvement of solubility was additionally confirmed. Specifically, solubility was confirmed as to how well the ceramide or the ceramide mixture dissolves in pure ethanol (94.5% or more).

As a result, as illustrated in Table 8, in a case of ceramide PC-104 alone, solubility was measured as about 0.04636 mg/uL, and, in a case of NPM, solubility was measured as about 0.00275 mg/uL. When ethanol solubility according to mixing of ceramide PC-104 and NPM was confirmed, an aspect in which ethanol solubility increases as a DSC enthalpy value is low was confirmed. Through this experiment, it was confirmed that an optimal mixing composition in terms of DSC measurement is excellent also in terms of ethanol solubility.

**[Table 8]**

| **Solubility analysis results according to mixing ratio of ceramide PC-104 and NPM** | | | |
|---|---|---|---|
| PC-104 | NPM | EtOH solubility (mg/uL) | DSC enthalpy (J/g) of corresponding composition |
| 10 | 0 | about 0.04636 | 151.6 |
| 9 | 1 | about 0.07000 | 4.9 |
| 2 | 8 | about 0.04320 | 36.8 |
| 1 | 9 | about 0.01667 | 44.0 |
| 0 | 10 | about 0.00275 | 45.3 |

Since an actual ethanol solubility by a PC-104:NPM = 0:10 mixing ratio (NPM alone) is about 0.00275 mg/uL, an actual ethanol solubility by a PC-104:NPM = 9:1 mixing ratio indicates a 25.5-fold solubility improvement as compared with an actual ethanol solubility by NPM alone use.

### [Example 3]

### Preparation of microparticles and lipid film sample

A lipid microparticle was manufactured by mixing stearic acid and cholesterol with a ceramide mixed in an optimal combination of PC-104 and NPM derived through a ceramide compatibility study. Particles were manufactured using a fluidized bed granulator (GPCG-1, Glatt, Germany), and particles were manufactured while spraying, ceramide in which PC-104 and NPM are mixed at 85 weight (wt)%:15 wt%, and mixed stearic acid (a raw material in a form in which myristic acid up to 2 wt%, palmitic acid 52 to 56 wt%, stearic acid 42 to 47 wt%, and lauric acid up to 1 wt% or less are mixed), and cholesterol after dissolving the same in a solvent such as ethanol. It was confirmed that microparticles (104NP-MP) in which PC-104 and NPM are mixed have a size at a level of 380 µm or less. It was confirmed that 104-MP made with PC-104 alone as a ceramide and with mixed stearic acid and cholesterol at a same ratio has a size at a similar level. A mixture in which the particles are dispersed in 0.15 wt% polyacrylic acid (Goldschmidt, Germany) was manufactured. A lipid film was manufactured by mixing ceramide (regarding, as a ceramide, a mixture in which PC-104 85 wt%:NPM 15 wt% are mixed) 49.37 wt%, stearic acid 49.37 wt%, and cholesterol 1.26 wt%, heating and dissolving, putting a dissolved solution into a mold so as to be a plate shape having a thickness of 2 mm or more, and cooling.

### [Example 4]

### Analysis of small-angle X-ray scattering (SAXS) and wide-angle X-ray scattering (WAXS)

In order to confirm a structural change shown when PC-104 and NPM are mixed, measurement of small-angle X-ray scattering (SAXS) and wide-angle X-ray scattering (WAXS) was performed. In SAXS, analysis of a lamellar structure of a lipid was able to be performed, and, in WAXS, analysis of a packing structure between lipids was able to be performed. Meanwhile, through comparison between samples, a lipid film (LF) is composed of only ceramide, a fatty acid, and cholesterol, thereby being able to confirm a structure that lipid compositions essentially prefer, and microparticles (MP) were able to confirm an internal structure that particles have in a state suitable for skin application (FIG. 3). First, in analysis using SAXS, in a lipid film (104-LF) including PC-104 as a ceramide, a lipid film (104NP-LF) including PC-104 and NPM as a ceramide, and microparticles (104-MP) including PC-104, it was confirmed that a short periodicity phase (SPP) structure may exist through an I peak (4.73 nm), whereas, only in microparticles (104NP-MP) in which PC-104 and NPM are mixed, peaks 1 and 2 (9.99, 4.96 nm) presumed to be a long periodicity phase (LPP) were confirmed. That is, the above indicates that an LPP structure may be generated in MP only when a ceramide is a 104+NPM mixture. In addition, while two peaks of * (5.20 nm, 4.07 nm) were confirmed in 104-LF, only a * peak (5.04 nm) was confirmed in 104NP-LF, and the above means that 104NP-LF forms a more uniform structure (FIG. 3C). Also in MP, as in LF, 104-MP shows three peaks of * (6.1 nm, 4.96 nm, 3.99 nm), but 104NP-MP shows two peaks of * (4.96 nm, 3.99 nm), thereby confirming a tendency in which structural uniformity is further increased (FIG. 3A).

Through WAXS measurement, in all four samples, H, O peaks (0.42 nm, 0.38 nm) capable of indicating a hexagonal and an orthorhombic structure were confirmed. However, in a case of 104NP, in LF, an O peak (0.36 nm or 0.37 nm) was weakly confirmed, but, in MP, an O peak (0.38 nm) was more strongly confirmed. The above meant that an orthorhombic structure may be strengthened more in MP. An orthorhombic packing exists dominantly in a normal temperature (30 to 32°C) of human skin, but there is a study in which, when a temperature of a stratum corneum is increased (30 to 40°C), conversion from an orthorhombic to a hexagonal lateral packing was observed (J. Bouwstra et al., The lipid organisation of the skin barrier: liquid and crystalline domains coexist in lamellar phases. Volume 28, pages 211-223, (2002)). Therefore, the results above suggest that, when MP is applied rather than LF, lipid components may be absorbed into skin in a structural form closer to normal skin.

### [Example 5]

### Confirmation of effect of increase of liquid crystals by ceramide mixing in oil-in-water emulsion

As in Table 9, an emulsion was made according to a comparative example (PC-104 alone) and an example (mixing PC-104 and NPM at 85 wt%:15 wt%) prescription, and the formation of liquid crystals was compared through a polarized microscope.

**[Table 9]**

| **Comparison of formation of liquid crystals according to ceramide mixing** | | |
|---|---|---|
| | Comparative Example | Example 5 |
| SQUALANE | 10 | 10 |
| ARACHIDYL ALCOHOL*BEHENYL | 2 | 2 |
| ALCOHOL*ARACHIDYL | | |
| GLUCOSIDE*GLUCOSE*WATER | | |
| PC-104 | 1.5 | 1.5 |
| NPM | - | 0.28 |
| WATER | 83.85 | 83.57 |
| HYDROXYETHYL ACRYLATE/SODIUM ACRYLOYLDIMETHYL TAURATE | 0.5 | 0.5 |
| COPOLYMER*SORBITAN | | |
| ISOSTEARATE*POLYSORBATE 60 | | |
| MIXED OF 3 KINDS OF SKIN CONDITIONING AGENTS | 2.15 | 2.15 |

As a result, as illustrated in FIG. 4, it was able to be confirmed that the formation of liquid crystals is increased in the example. Since it is known that the formation of liquid crystals in a general emulsion has various positive effects such as improvement of moisturizing power, it was able to be confirmed, also in an oil-in-water emulsion, an advantage when PC-104 and NPM are mixed and used as compared with when PC-104 is used alone.

The present disclosure is further described by the following implementations that do not limit the claims.

Implementation 1. A ceramide mixture including one or more kinds of ceramide NP; and PC-104 (hydroxypropyl bispalmitamide MEA).

Implementation 2. The ceramide mixture of Implementation 1, wherein the ceramide NP is ceramide NP to which a fatty acid having a carbon number of C14 to C24 is bound.

Implementation 3. The ceramide mixture of any one of Implementations 1 to 2, wherein the fatty acid includes one or more selected from the group consisting of palmitic acid, stearic acid, and oleic acid.

Implementation 4. The ceramide mixture of any one of Implementations 1 to 3, wherein the ceramide NP includes one or more selected from the group consisting of ceramide NP to which stearic acid is bound; ceramide NP to which oleic acid is bound; and mixed ceramide NP consisting of a plurality of ceramide NPs to which fatty acids having carbon numbers of C14 to C24 are bound.

Implementation 5. The ceramide mixture of any one of Implementations 1 to 4, including PC-104 and the ceramide NP at a weight ratio of 9.99:0.01 to 1:9.

Implementation 6. The ceramide mixture of any one of Implementations 1 to 5, wherein the ceramide NP is mixed ceramide NP consisting of a plurality of ceramide NPs to which fatty acids having carbon numbers of C14 to C24 are bound, and including PC-104 and the ceramide NP at a weight ratio of 9.99:0.01 to 3:7.

Implementation 7. The ceramide mixture of any one of Implementations 1 to 6, wherein the ceramide NP is ceramide NP to which oleic acid is bound, and including PC-104 and the ceramide NP at a weight ratio of 9.99:0.01 to 1:9.

Implementation 8. The ceramide mixture of any one of Implementations 1 to 7, wherein the ceramide NP is ceramide NP to which stearic acid is bound, and including PC-104 and the ceramide NP at a weight ratio of 9.99:0.01 to 2:8.

Implementation 9. A composition including the ceramide mixture of any one of Implementations 1 to 8.

Implementation 10. The composition of Implementation 9, further including ethanol as a solvent.

Implementation 11. The composition of any one of Implementations 9 to 10, further including a fatty acid and/or cholesterol.

Implementation 12. The composition of any one of Implementations 9 to 11, wherein the fatty acid is a saturated fatty acid having a carbon number of 10 to 30.

Implementation 13. The composition of any one of Implementations 9 to 12, wherein the composition has a form of a particle or a film.

Implementation 14. The composition of any one of Implementations 9 to 13, wherein the composition is an emulsion formulation.

Implementation 15. The composition of any one of Implementations 9 to 14, wherein the emulsion formulation is an oil-in-water emulsion (O/W emulsion) formulation.

Implementation 16. The composition of any one of Implementations 9 to 15, wherein the emulsion formulation has a liquid crystal structure.

Implementation 17. The composition of any one of Implementations 9 to 16, wherein the composition is a composition for skin moisturization; improvement of a skin moisture state; or prevention, improvement, or treatment of a skin disease.

## Claims

1. A ceramide mixture comprising one or more kinds of ceramide NP; and PC-104 (hydroxypropyl bispalmitamide MEA).

2. The ceramide mixture of claim 1, wherein the ceramide NP is ceramide NP to which a fatty acid having a carbon number of C14 to C24 is bound.

3. The ceramide mixture of claim 2, wherein the fatty acid comprises one or more selected from the group consisting of palmitic acid, stearic acid, and oleic acid.

4. The ceramide mixture of claim 2, wherein the ceramide NP comprises one or more selected from the group consisting of ceramide NP to which stearic acid is bound; ceramide NP to which oleic acid is bound; and mixed ceramide NP consisting of a plurality of ceramide NPs to which fatty acids having carbon numbers of C14 to C24 are bound.

5. The ceramide mixture of claim 1, comprising PC-104 and the ceramide NP at a weight ratio of 9.99:0.01 to 1:9.

6. The ceramide mixture of claim 5, wherein the ceramide NP is mixed ceramide NP consisting of a plurality of ceramide NPs to which fatty acids having carbon numbers of C14 to C24 are bound, and comprising PC-104 and the ceramide NP at a weight ratio of 9.99:0.01 to 3:7.

7. The ceramide mixture of claim 5, wherein the ceramide NP is ceramide NP to which oleic acid is bound, and comprising PC-104 and the ceramide NP at a weight ratio of 9.99:0.01 to 1:9.

8. The ceramide mixture of claim 5, wherein the ceramide NP is ceramide NP to which stearic acid is bound, and comprising PC-104 and the ceramide NP at a weight ratio of 9.99:0.01 to 2:8.

9. A composition comprising the ceramide mixture of any one of claims 1 to 8.

10. The composition of claim 9, further comprising ethanol as a solvent.

11. The composition of claim 10, further comprising a fatty acid and/or cholesterol.

12. The composition of claim 11, wherein the fatty acid is a saturated fatty acid having a carbon number of 10 to 30.

13. The composition of claim 9, wherein the composition has a form of a particle or a film.

14. The composition of claim 9, wherein the composition is an emulsion formulation.

15. The composition of claim 14, wherein the emulsion formulation is an oil-in-water emulsion (O/W emulsion) formulation.

16. The composition of claim 14, wherein the emulsion formulation has a liquid crystal structure.

17. The composition of claim 16, wherein the composition is a composition for skin moisturization; improvement of a skin moisture state; or prevention, improvement, or treatment of a skin disease.
